(19) Europäisches Patentamt
European Patent Office
Office européen des brevets

(11) Publication number: 0 086 510 B1

(12) **EUROPEAN PATENT SPECIFICATION**

(45) Date of publication of patent specification: 13.11.85

(51) Int. Cl.[4]: C 07 C 147/04, C 07 C 147/08, C 07 D 211/82, C 07 C 147/06

(21) Application number: 83200055.8

(22) Date of filing: 14.01.83

(54) **Process for the preparation of enamines; novel enamines and the use of enamines in the preparation of dihydropyridines.**

(30) Priority: 17.02.82 GB 8204595

(43) Date of publication of application: 24.08.83 Bulletin 83/34

(45) Publication of the grant of the patent: 13.11.85 Bulletin 85/46

(84) Designated Contracting States: BE DE FR GB NL

(56) References cited:
US-A-2 959 617
US-A-4 162 321
CHEMICAL ABSTRACTS, vol. 50, no. 1, January 10, 1956, column 221i-222c, COLUMBUS OHIO (US)
JOURNAL OF ORGANIC CHEMISTRY, vol. 31, November 1966, WASHINGTON D.C. (US), W.E. TRUCE et al.: "Stereochemistry of amine additions to acetylenic sulfones and carboxylic esters", pages 3543-3550
MONATSHEFTE FÜR CHEMIE, vol. 103, 1972, VIENNA (AT), H. STETTER et al.: "Zur Kenntnis der ungesättigten 1,1-Disulfone", pages 1262-1270

(73) Proprietor: SHELL INTERNATIONALE RESEARCH MAATSCHAPPIJ B.V.
Carel van Bylandtlaan 30
NL-2596 HR Den Haag (NL)

(72) Inventor: Feringa, Bernard Lucas
Badhuisweg 3
NL-1031 CM Amsterdam (NL)

(74) Representative: Aalbers, Onno et al
P.O. Box 302
NL-2501 CH The Hague (NL)

EP 0 086 510 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention provides a processs for the preparation of enamines, certain novel enamines and the use of enamines in the preparation of dihydropyridine-type compounds which may be of interest per se or as intermediates in the preparation of potential biologically active compounds.

The present invention thus relates to a process for the preparation of enamines according to the general formula

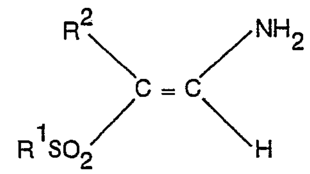
(I)

wherein $R^1$ represents an alkyl, aryl, alkaryl or aralkyl group, which may or may not be substituted by one or more inert substituents and $R^2$ represents a hydrogen or halogen atom, a group $R^1$ or a group $R^1SO_2$ or a cyano group, or $R^1$ and $R^2$ form part of a ring system containing at least three carbon atoms.

Several methods have been proposed in the art to prepare enamines according to the general formula I, which are reported to be stabilized by virtue of the presence of the sulphonyl group next to the carbon-carbon double bond. However, the syntheses described for such stabilized enamines require rather reactive and difficult to prepare starting materials such as phenylsulphonyl acetylenes [J. Org. Chem. *31* (1966) 3543] or bissulphonyl vinyl ethers [Monatsh. Chem. *103* (1972) 1262—1270]. It would be of interest to develop a process for the preparation of sulphonyl vinyl amines according to the general formula I from readily available or accesible starting materials. Thusfar, the use of nitriles as starting materials to produce enamines according to the general formula I has not been suggested at all since reduction of nitriles normally would lead to the production of the corresponding saturated amines.

Surprisingly, it has now been found that nitriles according to the general formula

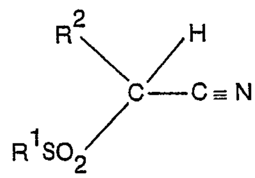
(II)

wherein $R^1$ and $R^2$ are as defined hereinbefore, can be used successfully as starting materials for the preparation of enamines according to the general formula I, even without the co-production of the expected saturated amines. Without wishing to be bound to any particular theory, it is believed that the initial reduction of the nitrile group in a compound according to the general formula II to the imine leads to a shift of the intermediate imine into the enamine form which is apparently sufficiently stabilized by the presence of the sulphonyl group to prevent further reduction.

The present invention thus relates to a process for the preparation of compounds according to the general formula

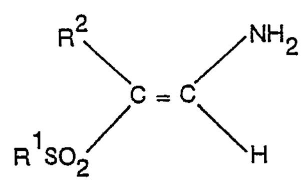
(I)

wherein $R^1$ represents an alkyl, aryl, alkaryl or aralkyl group, which may or may not be substituted by one or more inert substituents, and $R^2$ represents a hydrogen or halogen atom, a group $R^1$ or a group $R^1SO_2$ or a cyano group or $R^1$ and $R^2$ form part of a ring system containing at least three carbon atoms, characterised in that a compound according to the general formula

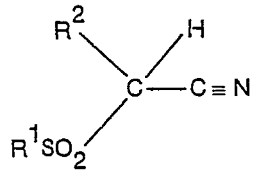
(II)

wherein $R^1$ and $R^2$ are as defined hereinbefore is reacted with a hydride-type reducing agent of the general formula

$$(M^1)_nM^2H_xR_y \qquad (III)$$

wherein $M^1$ represents a metal of Group I of the Periodic Table and $M^2$ represents an element of Groupe III of the Periodic Table, n is 0 or 1, R represents a hydrogen atom or one or more (dis)similar alkyl or alkoxy groups, and $x + y = 3$ when $n = 0$ or $x + y = 4$ when $n = 1$ provided that x is at least 1.

Examples of inert substituents in the formulas I and II comprise halogen atoms and haloalkyl, alkoxy, aryloxy or hydroxyalkyl groups such as chlorine, trifluoromethyl, methoxy, phenoxy and hydroxymethyl.

Examples of suitable reducing agents according to the general formula III comprise diborane, aluminium hydride, lithium aluminium hydride, sodium aluminium hydride, potassium aluminium hydride, lithium borohydride, sodium borohydride and potassium borohydride as well as the various alkyl or alkoxy substituted hydrides such as methyl lithium aluminium hydride, di-ethyl lithium aluminium hydride and sodium trimethoxy borohydride. Preference is given to the use of compounds according to the general formula III wherein $n = 1$ and $y = 0$, in particular to lithium aluminium hydride and sodium borohydride. Also complex reducing agents, i.e. reducing agents according to the general formula III, which additionally contain metal halides such as aluminium chloride or a titanium (IV) halide can be used.

It is highly remarkable that the reducing agents according to the general formula III do not reduce the enamines obtained any further into the corresponding saturated sulphonyl amines, not even diborane which is known to reduce enamines (vinyl amines) virtually quantitative.

The amount of reducing agent according to the general formula III to be used in the process according to the present invention is not critical and can vary between wide limits. Good results can be obtained using a nitrile: hydride-type compound molar ratio in the range of from 0.2:12, preference being given to the use of a molar ratio in the range of from 0.5:6. It has been found that the use of a rather large excess of hydride-type reducing agent exerts a beneficial effect on the yield of the desired sulphonyl vinyl amine (without coproduction of the corresponding saturated β-sulphonyl amine).

The process according to the present invention can be carried out conveniently at temperatures in the range of from 0°C to 100°C, preferably in the range of from 15° to 75°C.

If desired, the process according to the present invention can be carried out in the presence of an inert solvent. Examples of such solvents include ethers such as dimethylether, diethylether and n-dibutylether as well as cyclic ethers such as tetrahydrofuran and paraffins such as pentane, hexane, cyclohexane and isooctane. Good results have been obtained using tetrahydrofuran as a solvent. Also mixtures of solvents may be applied.

The process according to the present invention can be conveniently carried out batchwise. Good results have been obtained by adding a solution of the reducing agent, if desired in a (large) excess over the amount stoichiometrically required, to a solution of the nitrile to be converted, preferably under stirring of the reaction mixture. The reaction mixture can be worked up by methods known in the art. It may be convenient to quench the reaction mixture at a suitable moment with a lower alkanol, e.g. ethanol, prior to contacting the reaction mixture with water and a base in order to isolate the desired product.

The starting materials, i.e. the compounds according to the general formula II can be prepared by methods known in the art. A convenient method comprises the reaction between chloroacetonitrile and the appropraite quaternary ammonium sulphinate in tetrahydrofuran (Synthesis, 519, 1975). It is also possible to use a catalytic amount of a quaternary ammonium halide and an excess of the appropriate sulphinate (e.g. in the form of the sodium salt) in a suitable solvent (e.g. a mixture of water and methylene chloride).

Compounds according to the general formula I wherein $R^1$ represents an alkyl, aryl, alkaryl or aralkyl group, which may or not may be substituted by one or more inert substituents and $R^2$ represents a hydrogen atom or an alkyl, aryl, alkaryl or aralkyl group or $R^1$ and $R^2$ form part of a ring system containing at least four carbon atoms are believed to be novel compounds.

Preference is given to novel compounds according to the general formula I wherein $R^1$ represents an alkyl, aryl, alkaryl or aralkyl group having up to 20 carbon atoms which may or may not be substituted by one or more halogen, haloalkyl, alkoxy or aryloxy groups and $R^2$ represents a hydrogen atom or an alkyl or aryl group having up to 12 carbon atoms, in particular to compounds according to the general formula I, wherein $R^1$ represents an alkyl, aryl, alkaryl or aralkyl group having up to 10 carbon atoms, which may or may not be substituted by one or more halogen atoms or haloalkyl groups and $R^2$ represents a hydrogen atom or an alkyl or aryl group having up to 8 carbon atoms.

Examples of novel compounds according to the general formula I comprise β-methylsulphonyl vinyl amine, β-phenylsulphonyl vinyl amine, β-(p-tolylsulphonyl) vinyl amine and β-phenyl-β-(p-tolylsulphonyl) vinyl amine.

The enamines according to the general formula I can be suitably used as intermediates in various chemical reactions since they contain a rather strongly polarized carbon-carbon double bond. For instance, enamines according to the general formula I wherein $R^2$ represents a hydrogen atom can be used advantageously as starting materials for the synthesis of dihydropyridine type compounds according to the following reaction equation:

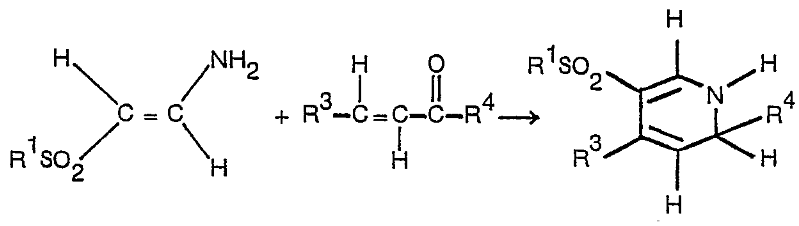

wherein $R^1$ is as defined hereinbefore and $R^3$ and $R^4$ which may be the same or different each represent an alkyl, aryl, aralkyl or alkaryl group which may or may not contain one or more inert substituents. Preference is given to the use of compounds according to the general formula I wherein $R^1$ represents an alkyl, aryl, alkaryl or aralkyl group having up to 10 carbon atoms, which may or may not be substituted by one or more halogen atoms or haloalkyl groups.

Unsaturated ketones as depicted hereinbefore which can be used advantageously in the reaction with the enamines comprise compounds wherein $R^3$ and $R^4$ are the same and both represent an alkyl or an aryl group. Good results for this type of Michael-condensations have been obtained using chalcone ($R^3 = R^4 =$ phenyl) as the starting material.

The process according to the reaction equation (1) can be carried out conveniently by adding a suspension of a strong base, e.g. sodium hydride or potassium-t-butylate in an ether such as tetrahydrofuran to the appropriate vinyl amine to which solution the unsaturated ketone, preferably in the same solvent, is added. The dihydropyridines can be isolated in good yields by methods known in the art.

The dihydropyridines according to the formula given in reaction equation (1) are believed to be novel compounds. Preferred compounds include those wherein $R^1$ represents an alkyl, aryl, alkaryl or aralkyl group having up to 20 carbon atoms which may or may not be substituted by one or more halogen, haloalkyl, alkoxy or aryloxy groups, and $R^3$ and $R^4$ which may be the same or different each represent an alkyl, aryl, aralkyl or alkaryl group which may or may not contain one or more inert substituents, in particular those compounds wherein $R^1$ represents an alkyl, aryl, alkaryl or aralkyl group having up to 10 carbon atoms, which may or may not be substituted by one or more halogen atoms or haloalkyl groups, and $R^3$ and $R^4$ are the same and both represent an alkyl or aryl group. Examples of dihydropyridine derivatives include 2,4-diphenyl-5-(p-tolylsulphonyl)-1,2-dihydropyridine and 2-methyl-4-phenyl-5-(p-tolylsulphonyl)-1,2-dihydropyridine. The dihydropyridine can be used advantageously in the synthesis of NADH-type (compounds nicotinamine-adenine nucleotides).

It is also possible to use the enamines according to the general formula I as starting materials for reactions with carbonyl compounds or epoxides. The present invention will now be illustrated by means of the following Examples.

## Example 1

Preparation of β-(p-tolylsulphonyl)-vinyl amine

a) Preparation of p-tolylsulphonyl methyl cyanide. Tetrabutylammonium-p-tolysulphinate (3.97 g) was added to chloroacetonitrile (0.76 g) in tetrahydrofuran (75 ml). The solution obtained was stirred at 30°C during 4.5 hours and subsequently poured into an aqueous solution of ammoniumchloride. The organic layer was separated and the aqueous layer was extracted with chloroform (2 × 75 ml). The combined organic layers were washed with water and dried over anhydrous $MgSO_4$. After removal of the solvent by distillation, a semi-solid was obtained. Crystallization from ethanol yielded p-tolylsulphonyl methyl cyanide (0.6 g; 30%).

b) To a solution of p-tolylsulphonyl methyl cyanide (0,195 g, 1 mmol) in anhydrous tetrahydrofuran (18 ml) was added lithium aluminium hydride (0,076 g; 2.0 mmol) dissolved in tetrahydrofuran. The resulting suspension was stirred during two hours at ambient temperature. Subsequently ethanol was added (5 ml) and the resulting mixture was poured into water (75 ml). Aqueous sodium hydroxide (15 ml of a 5% w solution) was added to this mixture under stirring. The organic layer obtained was separated and the aqueous layer was extracted with diethylether (2 × 30 ml). The combined organic layers were washed with a saturated aqueous solution of sodium chloride (30 ml) and dried over anhydrous $MgSO_4$. A semi-solid was obtained after removal of the solvent by a distillation treatment. The solid obtained contained β-(p-tolylsulphonyl)-vinyl amine (65% w) and p-tolylsulphonyl methyl cyanide (35% w). Crystallization from chloroform or methanol afforded pure β-(p-tolylsulphonyl)-vinyl amine as colourless needles, m.p. 92—94°C. The compound was characterized using proton nuclear magnetic resonance spectroscopy (δ, perdeuteromethanol) [2.54 (s, 3H, $CH_3$); 5.36 (d, 1H); 7.49 (d, 2H), 7.52 (d, 1H) and 7.85 (d, 2H)].

## Example 2

Preparation of β-phenylsulphonyl-vinyl amine

a) Preparation of phenylsulphonyl methyl cyanide. The experiment described in Example 1a was repeated using tetrabutylammonium-phenylsulphinate instead of the appropriate p-tolylsulphinate.

b) To a solution of phenylsulphonyl methyl cyanide (1,3 g) in tetrahydrofuran (45 ml) was added a six-fold molar excess of lithium aluminium hydride in tetrahydrofuran. The reaction mixture was worked-up as

described in Example 1b. β-Phenylsulphonyl-vinyl amine was obtained almost quantitatively as a semi-solid. Colourless crystals were obtained by crystallization from methanol. The compound was characterized using proton nuclear magnetic resonance spectroscopy (δ, perdeuteromethanol): 4.96 (2H); 5.38 (d, 1H) and 7.4—8.1 (m, 6H).

Example 3

Preparation of β-phenyl-β-(p-tolylsulphonyl)-vinyl amine

a) Preparation of 1-phenyl-p-tolylsulphonyl methyl cyanide. 1-Bromobenzylcyanide (prepared from 6.0 g benzylcyanide and 9.0 g bromide according to Organic Synthesis, coll. Vol. III, p. 347) was added to sodium p-tolylsulphinate (10 g) dissolved in a mixture of 96% ethanol (40 ml) and water (20 ml). The mixture obtained was refluxed during 45 minutes. The reaction mixture was filtered and the filtrate extracted with diethylether (2 × 50 ml). The combined organic layers were dried over anhydrous $MgSO_4$. After removal of the solvent by distillation 1-phenyl-p-tolylsulphonyl methyl cyanide was obtained in 45% yield (5.9 g). Colourless needles were obtained by crystallization from ethanol.

b) β-Phenyl-β-(p-tolylsulphonyl)-vinyl amine was prepared by the method as described in Example 2b (using a five-fold excess of lithium aluminium hydride) in 90% yield. Colourless crystals were obtained by crystallization from 96% ethanol. The compound was characterized using proton nuclear magnetic resonance spectroscopy (δ in deuterochloroform): 2.57 (s, 3H); 4.5 (2 × broad s, 2H) and 7.0—7.8 (m, 10H). The compound appears to be a mixture of the respective Z- and E isomers.

Example 4

The experiments described in Example 1 were slightly modified in order to obtain a higher yield of the product described. By using a catalytic amount of tetrabutylammoniumbromide (1 mole %) and an excess (2 to 5 fold) of sodium p-tolylsulphinate, chloroacetonitrile could be converted under phase-transfer conditions (using a water-methylene chloride 1:2 solvent mixture) into p-tolylsulphonyl methyl cyanide up to a yield of 60%.

β-(p-Tolylsulphonyl)-vinyl amine could be obtained in 95% yield by using a large excess of lithium aluminium hydride. Also longer reaction times and/or a higher reaction temperature contribute to better yields.

Example 5

Preparation of 2,4-diphenyl-5-(p-tolylsulphonyl)-1,2-dihydropyridine

To a suspension of sodium hydride (0.18 g, 7.5 mmol) in dry tetrahydrofuran (30 ml) was added β-(p-tolylsulphonyl)-vinyl amine (0.59 g, 3.0 mmol) under a nitrogen atmosphere. The resulting mixture was stirred at ambient temperature for 30 minutes. To the yellow solution obtained was added dropwise 0.62 g chalcone (3.0 mmol) in dry tetrahydrofuran (10 ml). The solution turned reddish-brown and was stirred at ambient temperature for 75 minutes. Thereafter, diethylether (20 ml) was added and the resulting mixture was subsequently poured into ice-cold water (100 ml). This mixture was acidified with aqueous hydrochloric acid to pH = 4 and subsequently neutralized using sodium carbonate. The organic layer was separated and the aqueous layer extracted with diethylether (20 × 30 ml). The combined organic layers were dried over anhydrous $MgSO_4$ and after removal of the solvent by distillation a white foaming solid was obtained. After crystallizated from benzene 2,4-diphenyl-5-(p-tolylsulphonyl)-vinyl amine was obtained in the form of colourless crystals, m.p. 164—166°C. The yield was about 60%. The compound was characterized using proton nuclear magnetic resonance spectroscopy (δ in deuterochloroform): 2.32 (s, 3H); 4.70 (d, 1H); 5.08 (d.d, 1H); 6.25 (broad d, 1H); 7.01 (d, 2H); 7.10 (s, 5H); 7.38 (s, 5H); 7.44 (d, 2H) and 7.66 (d, 1H).

Example 6

Preparation of 2-methyl-4-phenyl-5-(p-tolylsulphonyl)-1,2-dihydropyridine

β-(p-Tolylsulphonyl)-vinyl amine prepared according to the experiment described in Example 1b was reacted with 4-phenyl-3-buten-2-on according to the procedure described in the previous Experiment. A mixture of compounds was obtained containing as the major product 2-methyl-4-phenyl-5-(p-tolylsulphonyl)-1,2-dihydropyridine. The structure of this compound was assigned on the basis of comparison of chromatographic and proton nuclear magnetic resonance spectroscopic data for 2,4-diphenyl-5-(p-tolylsulphonyl)-1,2-dihydropyridine.

**Claims**

1. Process for the preparation of compounds according to the general formula

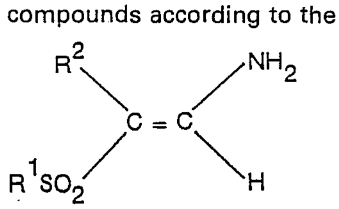

(I)

wherein $R^1$ represents an alkyl, aryl, alkaryl or aralkyl gorup, which may or may not be substituted by one or more inert substituents and $R^2$ represents a hydrogen or halogen atom, a group $R^1$ or a group $R^1SO_2$ or a cyano group, or $R^1$ and $R^2$ form part of a ring system containing at least three carbon atoms, characterized in that a compound according to the general formula

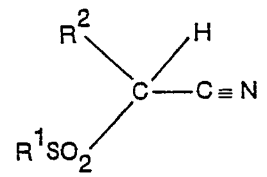

(II)

wherein $R^1$ and $R^2$ are as defined hereinbefore is reacted with a hydride-type reducing agent of the general formula

$$(M^1)_nM^2H_xR_y \quad \text{(III)}$$

wherein $M^1$ represents a metal of Group I of the Periodic Table and $M^2$ represents an element of Groupe III of the Periodic Table, n is 0 or 1, R represents a hydrogen atom or one or more (dis)similar alkyl or alkoxy groups, and $x + y = 3$ when $n = 0$ or $x + y = 4$ when $n = 1$ provided that x is at least 1.

2. Process according to claim 1, characterized in that a compound is used according to the general formula III wherein $n = 1$ and $y = 0$, in particular lithium aluminium hydride or sodium borohydride.

3. Process according to claim 1 or 2, characterized in that a compound according to the general formula II: hydride-type compound molar ratio in the range of from 0.2:12 is used, preferably in the range of from 0.5:6.

4. Process according to any of the preceding claims, characterized in that the reaction is carried out at a temperature in the range of from 0°C to 100°C, preferably in the range of from 15°C to 75°C.

5. Process according to any of the preceding claims, characterized in that an inert solvent, preferably a (cyclic) ether is used.

6. Compounds characterized by the general formula I, wherein $R^1$ represents an alkyl, aryl, alkaryl or aralkyl group, which may or may not be substituted by one or more inert substituents and $R^2$ represents a hydrogen atom or an alkyl, aryl, alkaryl or aralkyl group or $R^1$ and $R^2$ form part of a ring system containing at least four carbon atoms.

7. β-Phenylsulphonyl vinyl amine.

8. β-(p-Tolylsulphonyl) vinyl amine.

9. β-Phenyl-(p-tolylsulphonyl) vinyl amine.

**Patentansprüche**

1. Verfahren zur Herstellung von Verbindungen gemäß der allgemeinen Formel

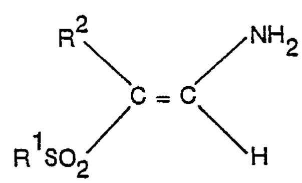

(I)

in welcher $R^1$ eine Alkyl-, Aryl, Alkaryl- oder Aralkylgruppe bedeutet, welche durch einen oder mehrere inerte(n) Substituenten substituiert sein kann oder nicht, und $R^2$ ein Wasserstoff- oder Halogenatom, eine Gruppe $R^1$, eine Gruppe $R^1SO_2$ oder eine Cyanogruppe darstellt, oder $R^1$ und $R^2$ einen Teil eines Ringsystems mit mindestens 3 Kohlenstoffatomen bilden, dadurch gekennzeichnet, daß eine Verbindung gemäß der allgemeinen Formel

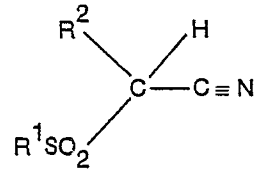

(II)

in welcher $R^1$ und $R^2$ wie oben definiert sind, mit einem hydridartigen Reduktionsmittel der Formel

$$(M^1)_nM^2H_xR_y \quad \text{(III)}$$

umgesetzt wird, in welcher $M^1$ ein Metall der Gruppe I des Periodensystems der Elemente darstellt und $M^2$

ein Element der Gruppe III des Periodensystems der Elemente ist, n 0 oder 1 bedeutet, R ein Wasserstoffatom oder eine oder mehrere (un)gleiche Alkyl- oder Alkoxygruppe(n) bedeutet, und $x + y = 3$ ist, wenn $n = 0$, oder $x + y = 4$ ist, wenn $n = 1$, mit der Maßgabe, daßx mindestens den Wert 1 hat.

2. Verfahren nach Anspruch 1, dadurch gekennzeichnet, daß eine Verbindung gemäß der allgemeinen Formel (III) verwendet wird, in welcher $n = 1$ und $y = 0$, insbesondere Lithium aluminiumhydrid oder Natriumborhydrid.

3. Verfahren nach Anspruch 1 oder 2, dadurch gekennzeichnet, daß eine Verbindung gemäß der allgemeinen Formel II in einem molaren Verhältnis zur hydridartigen Verbindung im Bereich von 0,2:12, vorzugsweise im Bereich 0,5:6 eingesetzt wird.

4. Verfahren nach einem der vorstehenden Ansprüche, dadurch gekennzeichnet, daß die Reaktion bei einer Temperatur im Bereich von 0°C bis 100°C, vorzugsweise im Bereich von 15°C bis 75°C durchgeführt wird.

5. Verfahren nach einem der vorstehenden Ansprüche, dadurch gekennzeichnet, daß ein inertes Lösungsmittel, vorzugsweise ein (zyklischer) Äther verwendet wird.

6. Verbindungen gekennzeichnet durch die allgemeine Formel I in welchen $R^1$ eine Alkyl-, Aryl-, Alkaryl- oder Aralkylgruppe darstellt, welche durch einen oder mehrere inerte Substituenten substituiert sein kann, oder nicht, und $R^2$ ein Wasserstoffatom oder eine Alkyl-, Aryl-, Alkaryl- oder Aralkyl-gruppe ist, oder $R^1$ und $R^2$ einen Teil eines Ringsystems, enthaltend mindestens 4 Kohlenstoffatome, bilden.

7. β-Phenylsulfonylvinylamin.

8. β-(p-Tolylsulfonyl)vinylamin.

9. β-Phenyl-(p-tolylsulfonyl)vinylamin.

**Revendications**

1. Procédé pour la préparation de composés de la formule générale

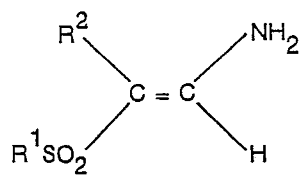

(I)

où $R^1$ représente un groupe alcoyle, aryle, alcaryle ou aralcoyle qui peut être non-substitué ou substitué par un ou plusieurs substituants inertes et $R^2$ représente un atome d'hydrogène ou d'un halogène, un groupe $R^1$ ou un groupe $R^1SO_2$ ou un groupe cyano, ou $R^1$ et $R^2$ font partie d'un système cyclique contenant au moins trois atomes de carbone, caractérisé en ce qu'un composé de la formule générale

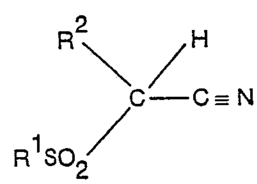

(II)

où $R^1$ et $R^2$ sont tels que définis ci-dessus est mis à réagir avec un agent réducteur de type hydrure de la formule générale

$$(M^1)_nM^2H_xR_y \qquad (III)$$

où $M^1$ représente un métal du groupe I du Tableau Périodique et $M^2$ représente un élément du groupe III du Tableau Périodique, n est 0 ou 1, R représente un atome d'hydrogène ou un ou plusieurs groupes alcoyle ou alcoxy identiques ou différents et $x + y = 3$ quand $n = 0$ ou $x + y = 4$ quand $n = 1$, avec la condition que x est au moins 1.

2. Procédé selon la revendication 1, caractérisé en ce qu'on utilise un composé de la formule générale III dans lequel $N = 1$ et $y = 0$, en particulier de l'hydrure de lithium et d'aluminium ou du borohydrure de sodium.

3. Procédé selon la revendication 1 ou 2, caractérisé en ce qu'on utilise un rapport molaire du composé de la formule générale II au composé du type hydrure compris entre 0,2 et 12, de préference entre 0,5 et 6.

4. Procédé selon l'une quelconque des revendications précédentes, caractérisé en ce que la réaction est conduite à une température comprise entre 0°C et 100°C, de préference entre 15°C et 75°C.

5. Procédé selon l'une quelconque des revendications précédentes, caractérisé en ce qu'on utilise un solvant inerte, de préférence un éther (cyclique).

6. Composés caractérisés par la formule générale I, où $R^1$ représente un groupe alcoyle, aryle, alcaryle ou aralcoyle, qui peut être non-substitué ou substitué par un ou plusieurs substituants inertes et $R^2$

représente un atome d'hydrogène ou un groupe alcoyle, aryle, alcaryle ou aralcoyle ou $R^1$ et $R^2$ font partie d'un système cyclique contenant au moins quatre atomes de carbone.

7. La bêta-phénylsulfonyl vinyl amine.
8. La bêta-(p-tolylsulfonyl) vinyl amine.
9. La bêta-phényl-(p-tolylsulfonyl) vinyl amine.